# EUROPEAN PATENT APPLICATION

(11) **EP 1 074 269 A2**
(43) Date of publication of application: **07.02.2001**
(21) Application number: 00306682.6
(22) Date of filing: 04.08.2000
(51) Int. Cl.: A61L 15/60

(54) **Disposable water-absorbent structure**

(30) Priority: 06.08.1999 JP 22331299
(71) Applicant: UNI-CHARM CORPORATION, Kawanoe-shi Ehime-ken (JP)
(72) Inventor: Onishi, Kazuaki, c/o Technical Center, Mitoyo-gun, Kagawa-ken 769-1602 (JP); Ishikawa, Norihiko, c/o Technical Center, Mitoyo-gun, Kagawa-ken 769-1602 (JP); Yabe, Youko, c/o Technical Center, Mitoyo-gun, Kagawa-ken 769-1602 (JP); Tatechou,Terumi, c/o Technical Center, Mitoyo-gun, Kagawa-ken 769-1602 (JP); Nagata, Manabu, c/o Sumitomo Seika Chem.Co.,Ltd., Shikama-ku, Himeji-shi, Hyogo 673-8076 (JP); Takemori, Shinichi, Sumitomo Seika Chem.Co.,Ltd., Shikama-ku, Himeji-shi, Hyogo 673-8076 (JP); Ueyama, Hiroyuki, Sumitomo Seika Chem.Co.,Ltd., Shikama-ku, Himeji-shi, Hyogo 673-8076 (JP)
(74) Representative: Parry, Christopher Stephen

(57) **Abstract**

A disposable water-absorbent structure 11 comprising highly water absorptive polymer particles 16, a fibrous support 17 and binders 18 disposed between the polymer particles 16 and the fibrous support 17. The fibrous support 17 is elastically compressive and bound to the polymer particles 16 by firmly binding effect and loosely binding effect, depending on spots in the structure 11. The structure thus formed prohibits the formation of gel block by water absorptive polymer particles possibly occurring in the structure.

## Description

This invention relates to a disposable water-absorbent structure suitable for absorption and disposal of body fluids, waste water or the like.

Water-absorbent structures of this type have already been proposed, for example, a structure comprising a mixture of highly water-absorptive polymer particles and fluff pulp compressed in a desired shape and entirely covered with tissue paper and the structure comprising highly water-absorptive polymer particles bound with a fibrous support by means of hot melt polymer particles. For example, Japanese Patent Application Disclosure Gazette No. 1994-245958 describes a water-absorbent structure comprising highly water-absorptive particles bound with the fibrous support by means of hot melt resin.

The structure of this type is suitable as absorbent cores of disposable diapers or sanitary napkins.

In general, it is essential that the polymer particles should be reliably held in the structure so far as the highly water-absorptive polymer particles are used as the component of the water-absorbent structure and the above-mentioned structure of prior art is an example taking a measure to meet this requirement.

However, the highly water-absorptive polymer particles tends to be bound together and consequently to form a gel block as they absorb water and swell, when an excessive amount of these polymer particles per unit area or unit volume. Once such gel block has been formed, the polymer particles confined within the block are no more utilized for water absorption and these relatively expensive polymer particles come to nothing since a water content of body fluids or the like can not permeate such gel block. Additionally, a water absorption of highly water-absorptive polymer particles is insufficient to obtain a water-absorbent structure having a high instantaneous water absorption merely by increasing an amount of the polymer particles. The above-mentioned document describes no measure to solve such problems of the assembly using the highly water-absorptive polymer particles.

It is an object of this invention to provide a water-absorbent structure using highly water absorptive polymer particles improved not only to alleviate formation of gel block by the polymer particles but also to realize a high instantaneous water absorption.

According to this invention, there is provided a water-absorbent structure comprising highly water absorptive polymer particles, a fibrous support and binders disposed between the polymer particles and the fibrous support to fix the polymer particles to the fibrous support, wherein:
the fibrous support is elastically compressive and firmly bound to the binders by a firmly binding effect, depending on spots in the fibrous support, in a manner that the fibrous support can not be easily dissociated from the binder even the polymer particles absorb water and swell and loosely bound to the binders, depending on spots in the fibrous support, in a manner that the fibrous support can be easily dissociated from the binders as the polymer particles absorb water and swell so that the fibrous support is maintained in compressed state thereof by the loosely binding effect.

With the water-absorbent structure of this invention comprising the highly water absorptive polymer particles, the staple fibers and the binders, the fiber interstices as well as the particle interstices are progressively enlarged as the polymer particles lying in the vicinity of the structure's upper surface absorb an amount of water and swell even if the polymer particles and the fibers are initially in compactly compressed state. As a result, the amount of water supplied onto the upper surface of the structure can permeate into the structure and be absorbed also by the polymer particles lying in a relatively deep region of the structure. In this manner, the structure of this invention enables most of the polymer particles to be successively utilized from those lying in the vicinity of the upper surface to those lying in the vicinity of the lower surface of the structure. The water-absorbent structure containing in its upper layer the hydrophilic fibers such as pulp fibers is particularly advantageous from the viewpoint of its ability of instantaneous water absorption.
Fig. 1 is a perspective view showing a partially cutaway disposable diaper using the structure according to this invention;
Fig. 2 is a sectional view taken along line II - II in Fig. 1;
Fig. 3 is a fragmentary diagram illustrating a composite assembly used by this invention in an enlarged scale;
Fig. 4 is a fragmentary diagram illustrating the water-absorbent structure according to this invention;
Fig. 5 is a view similar to Fig. 2 but showing one embodiment of this invention; and
Fig. 6 is a view similar to Fig. 2 but showing another embodiment of this invention.

Details of a water-absorbent structure according to this invention will be more fully understood from the description given hereunder with reference to the accompanying drawings.

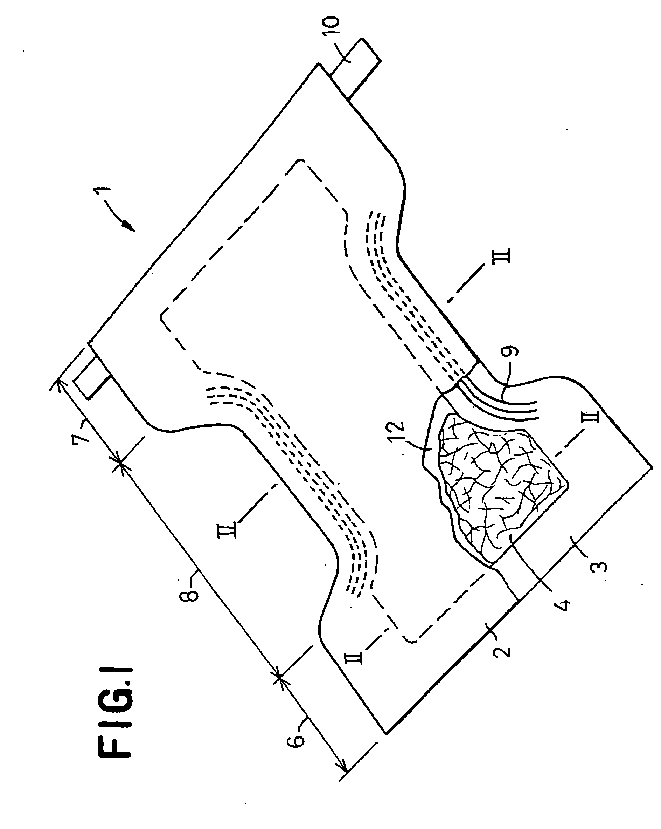

Fig. 1 is a perspective view showing a partially cutaway disposable diaper using the structure according to this invention. A diaper 1 comprises, as well known, a liquid-pervious topsheet 2, a liquid-impervious backsheet 3 and an absorbent core 4 disposed between these two sheets 2, 3. The diaper 1 is longitudinally composed of a front waist region 6, a rear waist region 7 and a crotch region 8 extending between these two waist regions 6, 7. The crotch region 8 has its transversely opposite side edges curved inwardly of the diaper 1. The diaper 1 is provided along the side edges of the crotch region 8 with elastic members 9 associated with a pair of leg-openings. The elastic members 9 are disposed between the topsheet 2 and the backsheet 3 and secured under tension to the inner surface of at least one of the topsheet 2 and the backsheet 3. Tape fasteners 10 laterally extend from transversely opposite side edges of the rear waist region 7, respectively.

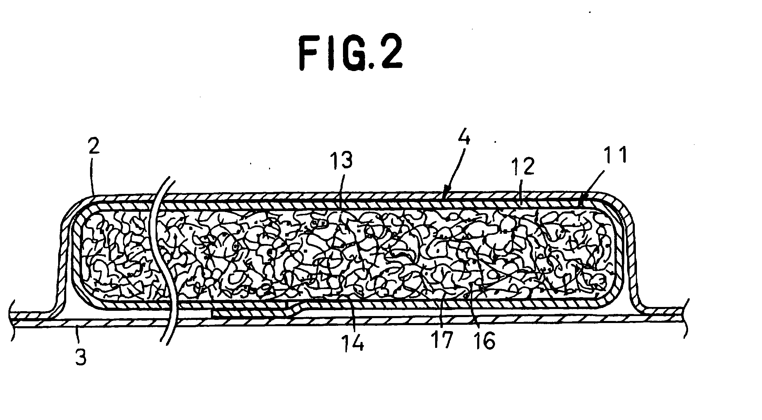

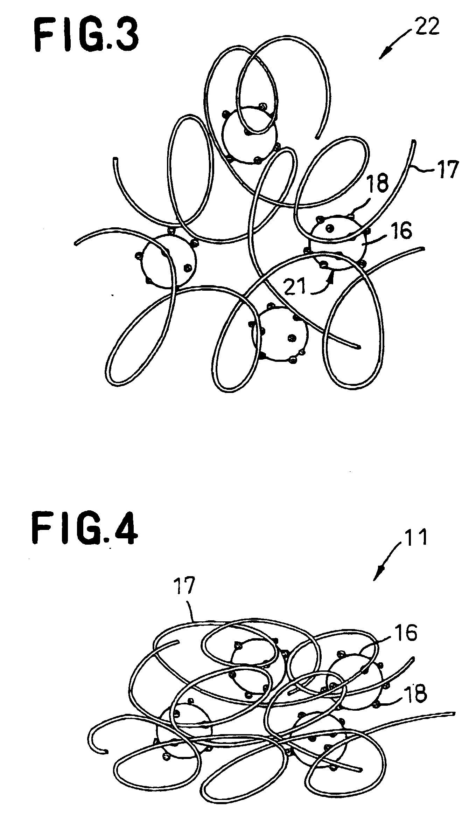

Fig. 2 is a sectional view taken along line II - II in Fig. 1. Referring to Fig. 2, the absorbent core 4 comprises the water-absorbent structure according to this invention exploited in the form of water-holding member 11 covered with a tissue paper 12. The water-holding member 11 is of a layer-like form having an upper surface 13 facing the diaper wearer's skin and a lower surface 14 opposed to the upper surface 13. The water-holding member 11 comprises highly water absorptive polymer particles 16, staple fibers 17 and binders 18 (See Figs. 3 and 4) functioning to fix the particles 16 to the fibers 17.

The highly water absorptive polymer particles 16 contained in thewater-holding member 11 are well known to those skilled in the art, for example, crosslinked acrylate polymer, crosslinked vinyl alcohol/acrylate copolymer, crosslinked graft polyvinyl alcohol maleic anhydride, crosslinked acrylate/methacrylate copolymer, crosslinked and saponified methacrylate/vinyl acetate copolymer, crosslinked starch/acrylate graft copolymer, crosslinked and saponified starch/acrylonitrile graft copolymer, crosslinked carboxymethyl cellulose, crosslinked isobutylene/maleate anhydride copolymer and crosslinked ethylene oxide polymer. One of these crosslinked polymers or copolymers or a mixture of two or more selected from them may be used as the polymer particles 16. An average particles diameter of these polymer particles is preferably in a range of 5 - 1000 µm and more preferably in a range of 100 - 600 µm.

The staple fibers 17 as the component of the water-holding member may contain crimped thermoplastic synthetic fiber of 100 - 40 % by weight and non-crimped thermoplastic synthetic fiber, natural fiber or chemical fiber of 0 - 60 % by weight. These thermoplastic synthetic fiber and chemical fiber preferably has a length of 5 - 100 mm and a fineness of 1 - 20 deniers. The crimped thermoplastic synthetic fiber is preferably conjugated fiber of side-by-side type or sheath-and-core type. The natural fiber and the chemical fiber are preferably of hydrophilic nature and fluff pulp may be used as the natural fiber and rayon fiber may be used as the chemical fiber. The staple fibers 17 are bonded and/or mechanically entangled together to form a layer-like form similar to the water-holding member 11. Within the water-holding member 11, the staple fibers 17 form a three dimensional support network adapted to support the polymer particles 16. The staple fibers 17 may be used in the form of nonwoven or woven fabric of in the form of fibrous assembly comprising the staple fibers 17 merely assembled at random. The crimped staple fibers 17 enables the water-holding member 11 to be elastically compressed in a thickness direction between the upper and lower surfaces 13, 14 and to elastically restore its initial thickness.

As the binders 18, hot melt polymer particles are used. These particles have a melting point preferably in a range of 50 - 150 °C, more preferably in a range of 60 - 130 °C and have an average particles diameter preferably in a range of 5 - 1000 µm, more preferably in a range of 100 - 600 µm. In soften or molten state, the binders 18 can be bound to both the polymer particles 16 and the staple fibers 17. Ethylene/glycidyl (metha) acrylate copolymer, one example of the polymer particles 25, is particularly advantageous since this copolymer can rapidly bind the highly water absorptive polymer particles 16 to the staple fibers 17. The expression "(metha) acrylate" used herein means both acrylate and methacrylate.

Specific examples of such ethylene/glycidyl (metha) acrylate copolymer include: ethylene/glycidyl acrylate copolymer, ethylene/glycidyl methacrylate copolymer, ethylene/glycidyl acrylate/glycidyl methacrylate copolymer, ethylene/glycidyl acrylate/vinyl acetate copolymer, ethylene/glycidyl acrylate/vinyl acetate/methyl acrylate copolymer, ethylene/glycidyl acrylate/methyl methacrylate copolymer, ethylene/glycidyl acrylate/vinyl acetate/methyl methacrylate copolymer, ethylene/glycidyl acrylate/ethyl acrylate copolymer, ethylene/glycidyl acrylate/vinyl acetate/ethyl acrylate copolymer, ethylene/glycidyl methacrylate/vinyl acetate copolymer, ethylene/glycidyl methacrylate/methyl acrylate copolymer, ethylene/glycidyl methacrylate/vinyl acetate/methyl acrylate copolymer, ethylene/glycidyl methacrylate/vinyl acetate/methyl methacrylate copolymer, ethylene/glycidyl methacrylate/methyl methacrylate copolymer, ethylene/glycidyl methacrylate/vinyl acetate/methyl methacrylate copolymer, ethylene/glycidyl methacrylate/ethyl acrylate copolymer, ethylene/glycidyl methacrylate/vinyl acetate/ethyl acrylate copolymer, ethylene/glycidyl acrylate/glycidyl methacrylate/vinyl acetate copolymer, ethylene/glycidyl acrylate/glycidyl methacrylate/methyl acrylate copolymer.

These copolymers may be used independently or in the form of a mixture. In order that the binders 18 can be evenly mixed with the polymer particles 16 under heating, these copolymers have a melt flow rate preferably in a range of 1 g/10 min - 400 g/10 min, more preferably in a range of 2 g/10 min - 100 g/10 min at 190 °C, 2160 gf.

Of the highly water absorptive polymer particles 16, the staple fibers 17 and the binders 18, the polymer particles 16 and the binders 18 may be mixed together preferably at a temperature at which the binders 18 is softened or molten, more preferably at a temperature at which the binders 18 is molten to form composite particles 21 (See Fig. 3) comprising the polymer particles 16 and the binders 18 firmly bound to the surfaces of the respective polymer particle 16. However, it must be avoided that the surface of each polymer particle 16 might be entirely covered with the binders 18. To avoid this, a weigh ratio of the binders 18 to the polymer particles is adjusted preferably at 0.5 - 50 : 100, more preferably at 3 - 30 : 100 or the particle diameter of the binder 18 is adjusted to an appropriate dimension so that preferably 5 - 50 %, more preferably 10 - 30 % of the surface of each polymer particle may be covered with the binder 18.

Now the composite particles 21 and the staple fibers 17 are mixed together with a weight ratio of 5 : 95 - 95 : 5 between the polymer particles 16 and the staple fibers 17 and then the mixture is heated at a temperature corresponding to or higher than the melting point of the binders 18. Alternatively, the composite particles 21 and the staple fibers 17 are mixed together at such temperature. Thereupon, the binders 18 in its molten or semi-molten state firmly binds the composite particles 21 to the staple fibers 17. In other words, the polymer particles 16 and the staple fibers 17 are bound together by means of the binders 18 to form a composite assembly 22 (See Fig. 3). The composite assembly 22 once formed is well resistant to its dissociation even when the polymer particles 21 absorb water and swell.

Fig. 3 is a fragmentary diagram illustrating the composite assembly 22 in an enlarged scale. Referring to Fig. 3, those having a relatively large dimension are the polymer particles 16 and those clinging to the surfaces of the respective polymer particles 16 are the particles of the binders 18. These large- and small-diametered particles form together the composite particles 21. The polymer particles 16 are fixed to the crimped staple fibers 17 by means of the binders 18. In the composite assembly 22, the staple fibers 17 are mechanically entangled one with another. Depending on the spots in the composite assembly 22, a single composite particle 21 is fixed to two or more staple fibers 17 and the staple fibers 17 are bonded one with another. With such construction, the crimped ones of the staple fibers 17 are elastically deformed as the composite assembly 22 is compressed and elastically restore their initial crimped states as the assembly 22 is relieved of the compressive force.

To obtain the water-holding member 11 from such composite assembly 22, the composite assembly of Fig. 3 may be developed to form a layer having a substantially uniform basis weight. Then this layer may be compressed under heating it at a temperature at which the binders 18 are softened or semi-molten until the staple fibers 17 are loosely bound to the binders 18 whereupon the layer may be cooled to maintain it in a compressed state.

Fig. 4 is a fragmentary diagram illustrating the water-holding member 11 obtained by compressing the composite assembly 22 of Fig. 3 under heating. The crimped staple fibers 17 are more remarkably collapsed than those in Fig. 3 and interstices among them, therefore, distances among the highly water absorptive polymer particles 16 also are closer than those in Fig. 3. An amount of water supplied to the upper surface 12 of the water-holding member 11 are absorbed by the polymer particles 16 and these polymer particles swell. The polymer particles 16 move as they swell and such movement of the polymer particles 16 causes the staple fibers 17 to move. As a result, the staple fibers 17 and the binders 18 loosely bound together during the process of compression are dissociated one from another so that the staple fibers 17 may elastically restore their crimped states as seen in Fig. 3 and spaced one from another. In this manner, the fibers 17 as well as the particles 16 are spaced one from another sufficiently to make undesired formation of gel block difficult. The amount of water supplied to the upper surface of the water-holding member 11 smoothly flows down through the interstices among the fibers 17 as well as among the particles 16 and is successively absorbed by the particles 16.

In the water-holding member 11, the elastic movement of the crimped staple fibers 17 prohibits formation of the gel block by the highly water-absorptive polymer particles 16 and these polymer particles 16 can be utilized successively from the upper surface toward the lower surface 14 of the water-holding member 11. In other words, it is not concerned that the polymer particles 16 might be more or less wastefully used. Such water-holding member 11 may be used as the component of the disposable diaper 1 to eliminate the problem that the amount of urine discharged on the diaper 1 might stay on the gel block formed by the polymer particles causing a leak of urine, stuffiness-and eruption. The compact water-holding member 11 having the staple fibers 17 maintained in their compressed states is well resistant to getting out of shape and not bulky. This feature facilitates the water-holding member 11 to be covered with tissue paper as shown in Fig. 1 and, in addition, facilitates the absorbent core 4 to be handled in the course of process for making the diaper 1. The diaper 1 itself as the final product is correspondingly compact and the space occupied by the diaper 1 in the course of distribution is effectively saved.

The staple fibers 17 are preferably mixed with suitable hydrophilic fibers such as pulp fibers or rayon fibers so that these mixed fibers may instantaneously absorb an amount of water and this amount of water once absorbed by said hydrophilic fibers may be then transferred to the highly water absorptive polymer particles 16. The water-holding member 11 of this arrangement is better than the water-holding member 11 in which all the staple fibers 17 are of synthetic fibers so far as the ability of instantaneous water absorption is concerned.

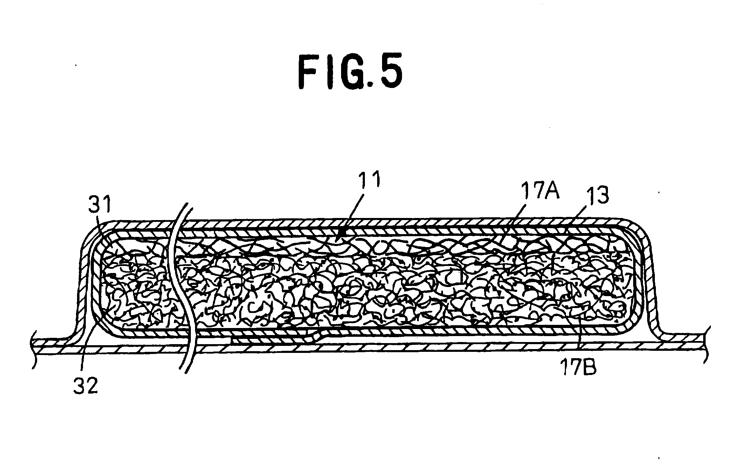

Fig. 5 is a view similar to Fig. 2 but showing one embodiment of this invention. The water-holding member 11 according to this embodiment has an upper layer 31 and a lower layer 32. The upper layer 31 is formed by thermoplastic synthetic resin staple fibers 17A having a relatively large fineness, for example, a fineness of 5 - 20 deniers, preferably of 8 - 15 deniers and may contain the component particle 21 corresponding to 0 - 5 % by weight of the staple fibers 17A. The lower layer 32 is formed by the staple fibers 17B having a fineness smaller than that of the staple fibers 17A in the upper layer 31, for example, a fineness of 1 - 10 deniers and may contain the highly water absorptive polymer particles 16 and the staple fibers 17B at a weight ratio of 5 : 95 - 95 : 5. The upper layer 31 has a thickness corresponding to 5 - 30 % and the lower layer 32 has a thickness corresponding to 95 - 70 % of the thickness of the water-holding member 11 in its compressed state. The staple fibers 17 as a whole inclusive of the staple fibers 17A, 17B in the upper and lower layers 31, 32 may contain the crimped thermoplastic synthetic fibers of 100 - 40 % by weight and non-crimped thermoplastic synthetic fibers, natural fibers and/or chemical fibers of 0 - 60 % by weight. While it is rather preferable to use the hydrophobic staple fibers 17A in the upper layer 31, it is possible to use the hydrophilic staple fibers in the upper layer 31. While it is rather preferable to use the hydrophilic staple fibers 17B in the lower layer 32, it is also possible to use the hydrophobic staple fibers in the lower layer.

The water-holding member 11 according to this embodiment is advantageous in that a substantially total amount of water permeates through the upper layer 31 to be absorbed by the lower layer 32 and therefore the upper surface 13 can be rapidly free from a wetness. Additionally, the staple fibers 17A of a relatively large fineness in the order of 8 - 15 deniers may be used in the upper layer 31 to facilitate the staple fibers 17 intertwined one with another to define relatively large interstices among them and, at the same time, to facilitate the lower layer 32 holding an amount of water to be adequately spaced from the diaper wearer's skin. In this manner, not only the water transfer from the upper layer 31 to the lower layer 32 is accelerated but also the highly water absorptive polymer particles 16 holding the amount of water are sufficiently spaced from the wearer's skin to protect the wearer from feeling the uncomfortable wetness.

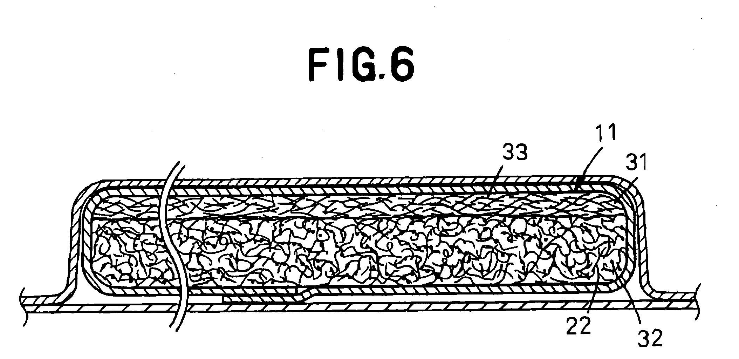

Fig. 6 also is a view similar to Fig. 2 but showing another embodiment of this invention. The water-holding member 11 according to this embodiment is formed by the hydrophilic upper layer 31 comprising pulp fibers 33 and the lower layer 32 comprising the composite assembly 22 in its compressed state as shown in Figs. 2 and 3. The upper layer 31 preferably has a thickness in the order of 5 - 30 % of the water-holding member 11 in its compressed state. This water-holding member 11 has its upper surface 13 softer than the upper surface 13 of the member 11 according to the embodiment shown in Fig. 2 and a high ability of instantaneous water absorption.

While the water-absorbent structure according to this invention has been described above with respect to the water-holding member 11 used to absorb body fluids, the water-absorbent structure of this invention is suitable as disposable articles rather than as repetitively used articles. This structure is applicable also to absorption of household or industrial waste water.

## Claims

1. A water-absorbent structure comprising highly water absorptive polymer particles, a fibrous support and binders disposed between said polymer particles and said fibrous support to fix said polymer particles to said fibrous support, wherein:
said fibrous support is elastically compressive and firmly bound to said binders by a firmly binding effect, depending on spots in said fibrous support, in a manner that said fibrous support can not be easily dissociated from said binders even said polymer particle absorb water and swell and loosely bound to said binders, depending on spots in said fibrous support, in a manner that said fibrous support can be easily dissociated from said binders as said polymer particles absorb water and swell so that said fibrous support is maintained in compressed state thereof by said loosely binding effect.

2. The structure according to Claim 1, wherein said fibrous support contains crimped conjugated fibers of 100 - 40 % by weight of said fibrous support.

3. The structure according to Claim 1, wherein said fibrous support is of a layer-like form having an upper surface and a lower surface and maintained in said compressed state between said upper and lower surfaces.

4. The structure according to Claim 1, wherein said fibrous support is formed by a nonwoven fabric comprising crimped conjugated fibers.

5. The structure according to Claim 3, wherein said fibrous support is at least two-layered network comprising an upper layer defining said upper surface and a lower layer underlying said upper layer and wherein said upper layer is formed by fibers having a fineness larger than that of fibers forming said lower layer.

6. The structure according to Claim 1, wherein said binders are ethylene/glycidyl (metha) acrylate copolymer.

7. The structure according to Claim 6, wherein said ethylene/glycidyl(metha)acrylate copolymer is at least one selected from a group consisting of ethylene/glycidyl acrylate copolymer, ethylene/glycidyl methacrylate copolymer, ethylene/glycidyl acrylate/vinyl acetate copolymer, ethylene/glycidyl methacrylate/vinyl acetate copolymer, ethylene/glycidyl acrylate/methyl acrylate copolymer, ethylene/glycidyl acrylate/ethyl acrylate copolymer, ethylene/glycidyl methacrylate/methyl acrylate copolymer.

8. The structure according to Claim 6, wherein a weight ratio of said ethylene/glycidyl(metha)acrylate copolymer to said highly water-absorptive polymer particles is 0.5 - 50 : 100.

9. The structure according to Claim 6, wherein said ethylene/glycidyl(metha)acrylate copolymer has a melting point of 50 - 150 °C.
